# EUROPEAN PATENT SPECIFICATION

(11) **EP 0 251 709 B1**
(45) Date of publication and mention of the grant of the patent: **21.04.1993**
(21) Application number: 87305670.9
(22) Date of filing: 25.06.1987
(51) Int. Cl.: G01N 33/50, G01N 33/66, C12Q 1/54

(54) **Agent for inhibiting glycolysis**
Wirkstoff zur Inhibierung von Glykolysis
Agent pour l'inhibition de la glycolyse

(30) Priority: 26.06.1986 JP 148036/86; 11.05.1987 JP 112289/87
(43) Date of publication of application: 07.01.1988
(73) Proprietor: IATRON LABORATORIES, INC., Chiyoda-ku Tokyo 101 (JP)
(72) Inventor: Nakashima, Koji, Edogawa-ku Tokyo (JP); Shibata, Hideto, Yotsukaido-shi Chiba (JP)
(74) Representative: Armitage, Ian Michael

(56) References cited:
- EP-A- 0 043 181
- WO-A-87/00769
- CLINICAL CHEMISTRY, vol. 33, no. 5, May 1987, Winston-Salem, NC (US); K.NAKASHIMA et al., pp. 708-710, Winston, Salem, US; K. NAKASHIMA et al.: "D-mannose as a preservative of glucose in blood samples"
- CHEMICAL ABSTRACTS, vol. 66, no. 7, 13 February 1967, Columbus, OH (US); E.BEUTLER et al., p. 2546, no. 27053u, Columbus, Ohio, US; E. BEUTLER et al.: "Blood preservation-relative capacities of hexoses, hexitols, and ethanol to maintain red cell ATP levels during storage"

## Description

The present invention relates to an inhibiting agent for a glycolysis of glucose in a blood sample taken for use in a clinical examination.

When a level of glucose in a blood sample is determined, an appropriate glycolysis inhibitor is generally added to the blood during or after drawing the blood, to inhibit a metabolism of glucose in the erythrocytes (red blood cells). This is because the presence of erythrocytes causes glucose to be metabolized as a nutriment thereof, and thus the level of glucose in the blood sample is reduced until the erythrocytes are removed or the determination is carried out. This brings about a considerable error in measurement results.

The metabolism mechanism of glucose is well known, and is caused by the actions of various enzymes in the erythrocytes. After sodium fluoride or monoiodoacetic acid salt, which are conventional agents widely used for glycolysis inhibition, is added to the blood sample, it enters the erythrocyte through the membrane thereof, inhibits enzymes such as phosphoglucomutase or enolase, thus inhibiting the metabolism of glucose, and controls the consumption of glucose in the blood sample.

Recently, however, it has been discovered that such conventional methods using sodium fluoride or monoiodoacetic acid salt do not always produce a complete inhibition of glycolysis and have a tendency to cause hemolysis, and thus are not always suitable for treating valuable blood samples (Program-References, pp. 306 - 317, for Fifth Summer Seminar by Rinsho Kagakukai, 1985).

Further, sodium fluoride or the like is not an inhibitor specific to enzymes belonging to the glycolytic pathway, but is a general inhibitor for enzymes. Therefore, in many cases, there is an adverse effect on other various routine examination items using blood samples, for example, an examination of the content of enzymes in the blood sample or of blood components by means of enzymes as reagents, and it becomes impossible to carry out such measurements during these examinations. Furthermore, in the usual determination of electrolytes, widely performed in a clinical examination, the addition of sodium fluoride makes it impossible to measure the levels of both sodium and potassium. This is because the addition of sodium per se influences the level of sodium in the blood sample, and because sodium fluoride or the like simultaneously inhibits any activity of the sodium-potassium-ATPase in the erythrocyte, and thus the equilibrium between potassium at a high concentration in the erythrocyte and sodium in the external liquid thereof is disturbed and an exudation of a large quantity of potassium to the external liquid occurs. This equilibrium is usually kept within a stationary range by the enzyme.

As explained above, when the level of glucose or metabolites thereof is determined in a clinical examination using a blood sample, a separate blood sample must be taken for this determination. Especially, the determination of the blood glucose usually requires a high examination frequency. This requires much work and is inefficient, and is also a burden to the blood donor, in particular a great mental and physical burden on an infant. In a glucose tolerance test frequently carried out during an examination for diabetes, the burden on the patient becomes greater because, after the glucose tolerance test, many blood samples must be taken successively, and these must be taken as two separate samples, i.e., one for determining glucose and the other for insulin, every time an examination is to be carried out.

The above disadvantage of an unsatisfactory inhibiting effect, economical drawbacks such as separate and frequent sampling and inefficiency, and above all, the mental and physical burden on the patient are considered to be serious problems in the taking of blood samples.

It is therefore desirable to provide a novel glycolysis inhibiting agent which is safe and simple, causes less damage such as hemolysis to the blood sample, and does not interfere in the results of a determination of almost all items other than the glucose level in the blood sample. International patent application WO 87/00769 published on 12 February 1987 discloses the use of mannose or derivatives thereof for inhibiting glycolsis in blood samples. This document is regarded as state of the art in accordance with Article 54(3) EPC.

In accordance with the present invention, there is provided an agent for inhibiting glycolysis of glucose in a blood sample, comprising D-mannosamine

In accordance with the present invention, there is also provided a method for inhibition of glycolysis of glucose in a blood sample, by employing D-mannosamine.

Further, in accordance with the present invention, there is provided a method for providing a blood sample for a determination of glucose levels therein, and the presence of electrolytes such as sodium, potassium, calcium, magnesium, chloride or inorganic phosphate, enzymes such as glutamic oxaloacetic transaminase (GOT), glutamic pyruvic transaminase (GPT), lactate dehydrogenase (LDH), alkaline phosphatase (ALP), acid phosphatase (ACP), amylase (AMY), cholinesterase (ChE), γ-glutamyl transpeptidase (γ-GTP), leucine aminopeptidase (LAP), creatine kinase (CK), or lipase, metals such as iron, copper, or zinc, biochemicals such as total protein (TP), albumin (Alb), blood urea nitrogen (BUN), creatinine, uric acid (UA), total bilirubin (T-Bil), triglyceride (TG), total cholesterol (T-CHO), phospholipid (PL), or free fatty acid (FFA), or a blood sample for various immunoassays, wherein D-mannosamine is incorporated in the blood sample.

Furthermore, in accordance with the present invention, there is provided a method for the inhibition of a glycolysis of glucose in a blood sample, by employing D-mannosamine in combination with at least one conventional agent (slow-effect agent) for inhibiting the glycolysis of glucose, such as sodium or potassium fluoride, or lithium or sodium monoiodoacetate.

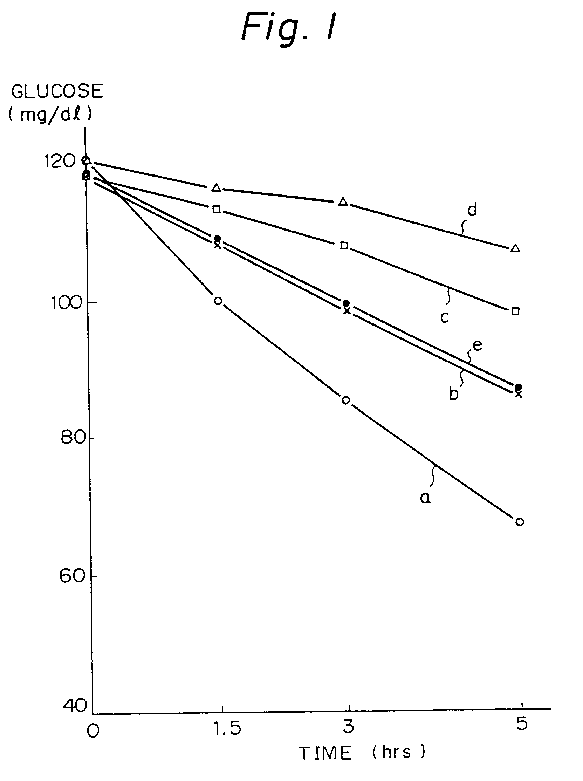

Figure 1 is a graph illustrating the glycolysis inhibiting effect of D-mannose and D-mannosamine.

According to the present invention, the agent for inhibiting the glycolysis of glucose comprises D-mannosamine. This inhibiting agent may be used alone or in combination with D-mannose and/or a derivative thereof. The derivative of D-mannose is a compound having a carbon skeleton and hydroxyl Xo$ stereo-configuration corresponding to those of D-mannose, wherein at least one of the hydroxyl groups is substituted by hydrogen, an amino or methoxy group, or phosphate, organic acid ester or amide.

The inhibiting agent according to the present invention has a rapid effect in comparison with the conventional inhibiting agents such as sodium fluoride and monoiodoacetic acid salt. The mechanism of the action by the inhibiting agent according to the present invention is not clearly understood, but it is believed to be essentially different from that of the conventional slow-effect agents.

The conventional inhibiting agents, as explained above, act by inhibiting the enzymes in the glucose metabolism, and thus the glucose metabolism is inhibited at a certain step in the metabolism process, after the glucose has entered the erythrocyte.

The inventors of the present invention carried out an investigation from a different viewpoint to that of the conventional inhibitors. More particularly, the inventors attempted various approaches to achieve the same purpose of prohibiting glucose from entering the erythrocyte through the membrane thereof. It is known that glucose is recognized by the receptor on the membrane of the erythrocyte, and introduced therein through the channel thereof. If this recognition of the glucose by the receptor could be blocked, the effect would be far more beneficial in comparison with the conventional inhibition, wherein glucose is led into the erythrocyte and glycolysis is inhibited therein. Such an approach has not been reported or published, and thus it was uncertain whether or not such approaches were even possible.

During the investigation along the above lines, the inventors surprisingly found that D-mannosamine exhibits the sought-after inhibiting effect, and can eliminate the disadvantages of the conventional inhibitors. It should be understood that the present inventors do not deny the possibility that the inhibiting agent of the present invention acts by inhibiting the enzymes in the glucose metabolism in the erythrocyte, similar to the conventional inhibitors.

The inhibiting agent according to the present invention may be added to the blood sample in the form of, for example, powder, a granule, a tablet, or the like, or as a solution of D-mannosamine.

The effect of the inhibiting agent according to the present invention is exerted regardless of the amount added, and therefore, there is no critical limitation to the amount of the inhibiting agent used in the present invention. However, preferably the inhibiting agent is added to the blood sample in the amount of 0.05 - 2.0 g/dl, more preferably 0.2 - 1 g/dl.

The inhibiting agent of the present invention may be variously applied, as long as a certain amount of the inhibiting agent coexists with the blood sample during or after the taking of the blood from the patient. For example, the blood is taken into a blood collection tube into which a certain amount of D-mannosamine has been previously charged. This is the simplest way, but there is no limit to the way in which this is done. Further, the inhibiting agent may be added in the form of a tablet immediately after the blood is taken.

As can be easily understood by those skilled in the art, the inhibiting agent of the present invention should not be used in a chemical determination of the glucose level wherein use is made of the reducing power of sugar, because D-mannosamine will cause serious errors in the results. However, enzymatic methods using hexokinase, glucose oxidase or the like are generally employed to improve the accuracy of the results of a determination of the glucose level in recent clinical-chemical examinations. In particular, the hexokinase method has been accepted internationally as a standard method. Therefore, it is desirable and advantageous to use D-mannosamine the derivative thereof as the glycolysis inhibiting agent in the enzymatic methods prevailing in this field.

The blood sample wherein the glycolysis of glucose is inhibited by adding D-mannosamine according to the present invention may be used to determine not only the level of glucose, but also the levels of electrolytes such as sodium, potassium, calcium, magnesium, chloride or inorganic phosphate, enzymes such as glutamic oxaloacetic transaminase (GOT) glutamic pyruvic transaminase (GPT), lactate dehydrogenase (LDH), alkaline phosphatase (ALP), acid phosphatase (ACP), amylase (AMY), cholinesterase (ChE), γ-glutamyl transpeptidase (γ-GTP), leucine aminopeptidase (LAP), creatine kinase (CK), or lipase, metals such as iron, copper, or zinc, biochemicals such as total protein (TP), albumin (Alb), blood urea nitrogen (BUN), creatinine, uric acid (UA), total bilirubin (T-Bil), triglyceride (TG), total cholesterol (T-CHO), phospholipid (PL), or free fatty acid (FFA). Further, such blood samples may be used for various immunoassays. This is because D-mannosamine does not affect the level of electrolytes, metals or biochemicals, or the activities of such enzymes or the like.

When D-mannosamine significantly interferes with the measurement results in any examination method, those skilled in the art will easily find an alternative conventional method to determine the same examination item.

If D-mannosamine is used in combination with the conventional inhibitors such as sodium fluoride, sodium monoiodoacetate or the like, the glycolysis of glucose in the blood sample can be rapidly and continuously inhibited. In this case, however, the blood sample can be used only for determining the level of glucose.

### EXAMPLE

The present invention now will be further illustrated by, but is by no means limited to, the following Example:

### Glycolysis Inhibiting Effect of D-Mannose and D-Mannosamine

D-Mannose was charged into heparinized tubes (Becton Dickinson & Co.) in amounts of 0 mg/dl, 50 mg/dl, 200 mg/dl, or 500 mg/dl, fresh blood was added thereto, respectively, and the tubes were left to stand at room temperature for 1.5, 3 or 5 hours, and then centrifuged. The amount of glucose in the sample was determined from the resulting plasma, using the glucose oxidase method, (Beckman Instruments Inc.) by an ASTFA 4 Autoanalyzer (Beckman Instruments Inc.). The results are shown in Figure 1, wherein symbols "a", "b", "c" and "d" denote the amounts of 0 mg/dl, 50 mg/dl, 200 mg/dl, and 500 mg/dl of D-mannose, respectively. A similar test was carried out using D-mannosamine in the amount of 300 mg/dl. The result obtained is also shown in Figure 1 (symbol "e").

As explained above, the glycolysis inhibiting agent according to the present invention comprises D-mannosamine, and thus does not have the strong inhibition effect on enzymes generally observed when using sodium fluoride or the like. Therefore, the inhibiting agent of the present invention may be used at a high concentration for inhibiting glycolysis. Further, the blood sample can be also used for determining electrolytes including sodium and potassium and other items including glucose in a usual clinical chemical examination, without any significant interference in the results thereof. The inhibiting agent according to the present invention is very safe, and rarely causes hemolysis of the blood sample. Accordingly, the present invention ameliorates the problems of the conventional glycolysis inhibitors, i.e., frequent operation, etc., in the determination of glucose levels or a glucose tolerance test, and above all, reduces the mental and physical burden on the patient.

## Claims

1. A method for inhibition of glycolysis of glucose in a blood sample, characterised by employing D-mannosamine.

2. A method according to claim 1 wherein the amount of D-mannosamine used ranges from 0.05 to 2.0 g/dl in the blood sample.

3. A method for providing a blood sample for a determination of levels therein of glucose, electrolytes, enzymes, metals or biochemicals, or for an immunoassay, characterised in that D-mannosamine is incorporated in the blood sample.

4. A method according to claim 3, wherein the amount of D-mannosamine used ranges from 0.05 to 2.0 g/dl in the blood sample.

5. A method for inhibition of glycolysis of glucose in a blood sample, characterised by employing D-mannosamine in combination with at least one conventional agent for inhibiting glycolysis of glucose.

6. A method according to claim 5, wherein the conventional inhibiting agent is sodium or potassium fluoride, or monoiodoacetic acid salt.

7. Use of D-mannosamine in the manufacture of an agent for inhibiting glycolysis of glucose in a blood sample.

8. An agent for inhibiting glycolysis of glucose in a blood sample comprising D-mannosamine in combination with at least one conventional agent for inhibiting glycolysis of glucose.

9. A blood sample containing added D-mannosamine.

## Patentansprüche

1. Verfahren zur Inhibierung der Glykolyse von Glucose in einer Blutprobe, dadurch gekennzeichnet, daß man D-Mannosamin verwendet.

2. Verfahren nach Anspruch 1, bei dem die Menge des verwendeten D-Mannosamins in der Blutprobe in einem Bereich von 0,05 bis 2,0 g/dl liegt.

3. Verfahren zum Vorsehen einer Blutprobe für die Bestimmung der Anteile von Glucose, Elektrolyten, Enzymen, Metallen oder Biochemikalen oder für eine Immununtersuchung, dadurch gekennzeichnet, daß der Blutprobe D-Mannosamin zugesetzt wird.

4. Verfahren nach Anspruch 3, bei dem die Menge des verwendeten D-Mannosamins in der Blutprobe in einem Bereich von 0,05 bis 2,0 g/dl liegt.

5. Verfahren zum Inhibieren der Glykolyse von Glucose in einer Blutprobe, dadurch gekennzeichnet, daß man D-Mannosamin in Verbindung mit mindestens einem herkömmlichen Wirkstoff zur Inhibierung der Glykolyse von Glucose verwendet.

6. Verfahren nach Anspruch 5, bei dem der herkömmliche Inhibierungswirkstoff Natrium- oder Kaliumfluorid oder Monojodessigsäuresalz ist.

7. Verwendung von D-Mannosamin bei der Herstellung eines Wirkstoffes zur Inhibierung der Glykolyse von Glucose in einer Blutprobe.

8. Wirkstoff zur Inhibierung der Glykolyse von Glucose in einer Blutprobe, der D-Mannosamin in Verbindung mit mindestens einem herkömmlichen Wirkstoff zur Inhibierung der Glykolyse von Glucose enthält.

9. Blutprobe, der D-Mannosamin zugesetzt ist.

## Revendications

1. Procédé pour l'inhibition de la glycolyse du glucose dans un échantillon sanguin, caractérisé en ce qu'on emploie de la D-mannosamine.

2. Procédé selon la revendication 1, dans lequel la quantité de D-mannosamine utilisée se situe entre 0,05 et 2,0 g/dl dans l'échantillon sanguin.

3. Procédé pour fournir un échantillon sanguin pour y déterminer les teneurs en glucose, électrolytes ou enzymes, métaux ou agents biochimiques, ou pour un immunodosage, caractérisé en ce qu'on incorpore de la D-mannosamine dans l'échantillon sanguin.

4. Procédé selon la revendication 3, dans lequel la quantité de D-mannosamine utilisée se situe entre 0,05 et 2,0 g/dl dans l'échantillon sanguin.

5. Procédé pour l'inhibition de la glycolyse du glucose dans un échantillon sanguin, caractérisé en ce qu'on emploie la D-mannosamine en combinaison avec au moins un agent classique pour inhiber la glycolyse du glucose.

6. Procédé selon la revendication 5, dans lequel l'agent inhibiteur classique est le fluorure de sodium ou de potassium, ou un sel de l'acide monoiodoacétique.

7. Utilisation de la D-mannosamine dans la préparation d'un agent pour inhiber la glycolyse du glucose dans un échantillon sanguin.

8. Agent pour inhiber la glycolyse du glucose dans un échantillon Zanguin comprenant la D-mannosamine en combinaison avec au moins un agent classique pour inhiber la glycolyse du glucose.

9. Echantillon sanguin contenant de la D-mannosamine ajoutée.
